# EUROPEAN PATENT APPLICATION

(11) **EP 2 980 180 A1**
(43) Date of publication of application: **03.02.2016**
(21) Application number: 14773431.3
(22) Date of filing: 26.02.2014
(51) Int. Cl.: C09K 3/00, C07C 211/62, C07D 233/58, C07D 233/61, C07D 487/04, C07F 5/02, C08G 59/68

(54) **PHOTOBASE GENERATOR**

(30) Priority: 28.03.2013 JP 2013068722
(71) Applicant: San-Apro Ltd., Kyoto-shi, Kyoto 605-0995 (JP)
(72) Inventor: IKEDA, Takuya, Kyoto-shi Kyoto 605-0995 (JP); SHIRAISHI, Atsushi, Kyoto-shi Kyoto 605-0995 (JP); FURUTA, Takeshi, Kyoto-shi Kyoto 605-0995 (JP)
(74) Representative: Kotitschke & Heurung Partnerschaft mbB
(86) International application number: PCT/JP2014/001003
(87) International publication number: WO 2014/155960

(57) **Abstract**

Provided are: a photobase generator which has higher sensitivity to light compared with conventional photobase generators and can exert a high effect when used in combination with a sensitizer; and a photosensitive resin composition which contains the photobase generator. The present invention is a photobase generator characterized by comprising an ammonium salt represented by general formula (1). 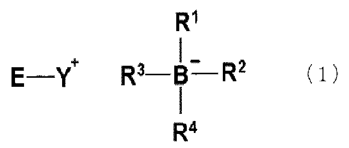 [In formula (1), R¹ to R⁴ independently represent an alkyl group having 1 to 18 carbon atoms or Ar, wherein at least one of R¹ to R⁴ represents Ar; Ar represents an aryl group having 6 to 14 carbon atoms (excluding carbon atoms contained in a substituent as mentioned below), wherein some of hydrogen atoms in the aryl group may be independently substituted by an alkyl group having 1 to 18 carbon atoms or the like; Y⁺ represents an ammonio group represented by general formula (2) or (3); and E represents a hydrogen atom or a group represented by general formula (5).]

## Description

### TECHNICAL FIELD

The present invention relates to a photobase generator generating a base by photoirradiation. More particularly, the present invention relates to a photobase generator suitably used for the production of a material to be cured by utilizing a base generated by photoirradiation (for example, a coating agent or a coating material) or a product or a member formed after being subjected to patterning in which the difference in solubility to a developing solution between the exposed part and the unexposed part is utilized (for example, an electronic component, an optical product, a forming material for an optical component, a layer forming material or an adhesive agent).

### BACKGROUND ART

As a photobase generator generating a base by being subjected to exposure, there have been known various photobase generators such as photobase generators generating primary amines or secondary amines (Patent Document 1 and Non-Patent Document 1) and photobase generators generating a strong base (tertiary amines, pKa 8 to 11) and a super-strong base (guanidine, amidine and the like, pKa 11 to 13) (Patent Documents 2 to 5, Non-Patent Document 2 and the like).

However, with regard to the photobase generators described in Patent Document 1 and Non-Patent Document 1, the basicity of the base generated is low (pKa < 8), and the photobase generator is not suitable as the catalyst for a polymerization reaction or for a crosslinking reaction because the activity is low. Moreover, since these amines have an active hydrogen atom, there has been a problem that a large amount of the photobase generator is required for allowing the reaction to proceed sufficiently because these amines themselves react with one another when used in a polymerization reaction or a crosslinking reaction of epoxides and isocyanates.

Moreover, the base generators described in Patent Documents 2 to 5 and Non-Patent Document 2 and the like have a problem that the performance is low when used as photo-latent base catalysts in a polymerization reaction or a crosslinking reaction of epoxides and isocyanates since the activity to light is low, and moreover, the base generator exerts a low effect when used in combination with a photosensitizer.

Under such circumstances, there has been desired the development of a photobase generator having a catalytic activity for sufficiently curing an epoxy resin, namely, a photobase generator in which the sensitivity to light is more enhanced compared with conventional photobase generators.

### PRIOR ART DOCUMENTS

### PATENT DOCUMENTS

Patent Document 1: JP-A-H10-7709
Patent Document 2: JP-A-2005-107235
Patent Document 3: JP-A-2005-264156
Patent Document 4: JP-A-2007-119766
Patent Document 5: JP-A-2009-280785
Patent Document 6: WO 2005/014696 A
Patent Document 7: WO 2009/122664 A

### NON-PATENT DOCUMENTS

Non-Patent Document 1: Dictionary of Optical Application Technologies and Materials, published by Sangyo-Gijutsu Service Center Co., Ltd., 2006, p. 130
Non-Patent Document 2: J. Photopolym. Sci. Tech., Vol. 19., No. 1 (81) 2006

### SUMMARY OF THE INVENTION

### PROBLEMS TO BE SOLVED BY THE INVENTION

The problem to be solved by the present invention is to provide a photobase generator which has higher sensitivity to light compared with conventional photobase generators, and moreover, can exert a great effect when used in combination with a sensitizer, and a photosensitive resin composition which contains the base generator.

### SOLUTIONS TO THE PROBLEMS

The present inventors have conducted intensive researches in view of solving said problems, and as a result, have found a photobase generator having excellent characteristics.

That is, the present invention is directed to a photobase generator comprising an ammonium salt represented by general formula (1).

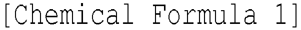

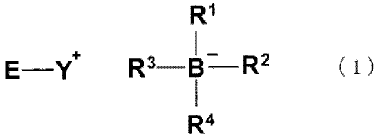

[In formula (1), R¹ to R⁴ independently represent an alkyl group having 1 to 18 carbon atoms or Ar, wherein at least one of R¹ to R⁴ represents Ar, Ar represents an aryl group having 6 to 14 carbon atoms (excluding carbon atoms contained in a substituent as mentioned below), some of hydrogen atoms in the aryl group may be independently substituted by an alkyl group having 1 to 18 carbon atoms, an alkenyl group having 2 to 18 carbon atoms, an alkynyl group having 2 to 18 carbon atoms, an aryl group having 6 to 14 carbon atoms, a nitro group, a hydroxyl group, a cyano group, an alkoxy group or aryloxy group represented by -OR²¹, an acyl group represented by R²²CO- , an acyloxy group represented by R²³COO-, an alkylthio group or arylthio group represented by -SR²⁴, an amino group represented by -NR²⁵R²⁶, or a halogen atom, R²¹ to R²⁴ independently represent an alkyl group having 1 to 8 carbon atoms or an aryl group having 6 to 14 carbon atoms, and R²⁵ and R²⁶ independently represent a hydrogen atom, an alkyl group having 1 to 8 carbon atoms or an aryl group having 6 to 14 carbon atoms; Y⁺ represents an ammonio group represented by general formula (2) or (3) below; in formula (2), R⁵ and R⁶ independently represent an alkyl group having 1 to 18 carbon atoms, an alkenyl group having 2 to 18 carbon atoms or an aryl group having 6 to 14 carbon atoms, and may be bonded to each other to form a ring structure; Q represents a methylene group (-CH₂-)m, or a group represented by general formula (4) below, and m represents an integer of 2 or 3; in formula (3), R⁷ to R⁹ independently represent an alkyl group having 1 to 18 carbon atoms, an alkenyl group having 2 to 18 carbon atoms or an aryl group having 6 to 14 carbon atoms, and may be bonded to one another to have a ring structure; in formula (4), R¹⁰ to R¹¹ independently represent a hydrogen atom, an alkyl group having 1 to 18 carbon atoms or an aryl group having 6 to 14 carbon atoms, and may be bonded to each other to form a ring structure; and E represents a hydrogen atom or a group represented by general formula (5) below, R¹² to R¹⁶ independently represent an alkyl group having 1 to 18 carbon atoms, a nitro group, a hydroxyl group, a cyano group, an alkoxy group represented by -OR²¹, an acyl group represented by R²²CO-, an acyloxy group represented by R²³COO-, an alkylthio group represented by -SR²⁴, an amino group represented by -NR²⁵R²⁶ or a halogen atom, R¹² to R¹⁶ may be the same as or different from one another, and R¹⁷ and R¹⁸ independently represent a hydrogen atom or an alkyl group having 1 to 18 carbon atoms, and may be the same as or different from each other.]

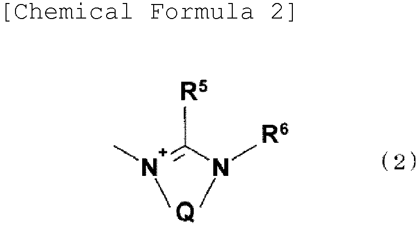

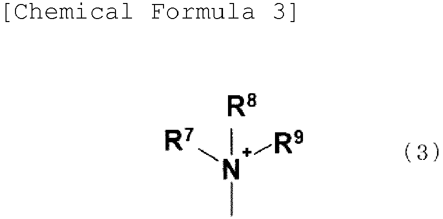

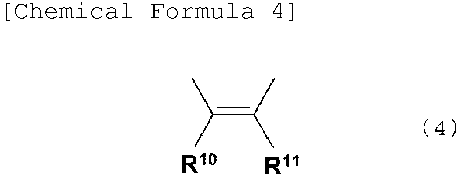

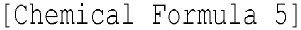

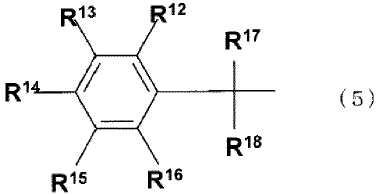

Furthermore, the present invention is directed to the photobase generator further comprising a photosensitizer as an active ingredient in addition to the above-described ammonium salt.

Furthermore, the present invention is directed to a photocurable composition comprising the above-described photobase generator and a basic reactive compound.

Furthermore, the present invention is directed to a cured product obtained by curing the above-mentioned photocurable composition.

### EFFECTS OF THE INVENTION

The photobase generator according to the present invention is sensitive to light and is capable of efficiently generating amines (tertiary amines and amidine) which are high in catalytic activity.

Moreover, the photobase generator according to the present invention is capable of more efficiently generating amines (tertiary amines and amidine) which are high in catalytic activity since the photobase generator can exert a higher effect when used in combination with a sensitizer compared with conventional photobase generators.

Moreover, since the photobase generator according to the present invention does not contain halogen ions and the like as counter anions, there is no fear of metal corrosion.

Moreover, since the photobase generator according to the present invention has no basicity before subjected to exposure, even when contained in a reactive composition, there is no possibility that the storage stability of the reactive composition is lowered.

Moreover, the photobase generator according to the present invention is stable also against heat and hardly generates a base even when heated as long as the photobase generator is not irradiated with light.

Moreover, according to a production method of a cured material prepared with the photocurable composition according to the present invention, by using the above-mentioned photobase generator and irradiating the photobase generator with light, it is possible to efficiently generate amines (tertiary amines and amidine) which are high in catalytic activity and to efficiently produce a cured material.

### MODE FOR CARRYING OUT THE INVENTION

Hereinafter, an embodiment of the present invention will be described in detail.

A photobase generator refers to such a substance that its chemical structure is decomposed by photoirradiation, so that it generates a base (amine). The base generated is capable of acting as a catalyst for a curing reaction of an epoxy resin, a curing reaction of a polyimide resin, a urethanization reaction between an isocyanate and a polyol, a crosslinking reaction of an acrylate, and the like.

A photobase generator according to the present invention is characterized by containing an ammonium salt represented by general formula (1).

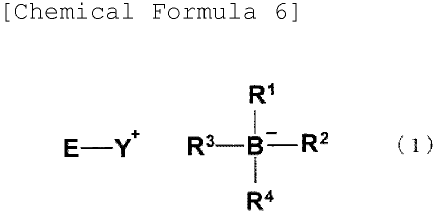

[In formula (1), R¹ to R⁴ independently represent an alkyl group having 1 to 18 carbon atoms or Ar, wherein at least one of R¹ to R⁴ represents Ar, Ar represents an aryl group having 6 to 14 carbon atoms (excluding carbon atoms contained in a substituent as mentioned below), some of hydrogen atoms in the aryl group may be independently substituted by an alkyl group having 1 to 18 carbon atoms, an alkenyl group having 2 to 18 carbon atoms, an alkynyl group having 2 to 18 carbon atoms, an aryl group having 6 to 14 carbon atoms, a nitro group, a hydroxyl group, a cyano group, an alkoxy group or aryloxy group represented by -OR²¹, an acyl group represented by R²²CO- , an acyloxy group represented by R²³COO-, an alkylthio group or arylthio group represented by -SR²⁴, an amino group represented by -NR²⁵R²⁶, or a halogen atom, R²¹ to R²⁴ independently represent an alkyl group having 1 to 8 carbon atoms or an aryl group having 6 to 14 carbon atoms, and R²⁵ and R²⁶ independently represent a hydrogen atom, an alkyl group having 1 to 8 carbon atoms or an aryl group having 6 to 14 carbon atoms; Y⁺ represents an ammonio group represented by general formula (2) or (3) below; in formula (2), R⁵ and R⁶ independently represent an alkyl group having 1 to 18 carbon atoms, an alkenyl group having 2 to 18 carbon atoms or an aryl group having 6 to 14 carbon atoms, and may be bonded to each other to form a ring structure; Q represents a methylene group (-CH₂-)m, or a group represented by general formula (4) below, and m represents an integer of 2 or 3; in formula (3), R⁷ to R⁹ independently represent an alkyl group having 1 to 18 carbon atoms, an alkenyl group having 2 to 18 carbon atoms or an aryl group having 6 to 14 carbon atoms, and may be bonded to one another to have a ring structure; in formula (4), R¹⁰ to R¹¹ independently represent a hydrogen atom, an alkyl group having 1 to 18 carbon atoms or an aryl group having 6 to 14 carbon atoms, and may be bonded to each other to form a ring structure; and E represents a hydrogen atom or a group represented by general formula (5) below, R¹² to R¹⁶ independently represent an alkyl group having 1 to 18 carbon atoms, a nitro group, a hydroxyl group, a cyano group, an alkoxy group represented by -OR²¹, an acyl group represented by R²²CO- , an acyloxy group represented by R²³COO-, an alkylthio group represented by -SR²⁴, an amino group represented by -NR²⁵R²⁶ or a halogen atom, R¹² to R¹⁶ may be the same as or different from one another, and R¹⁷ and R¹⁸ independently represent a hydrogen atom or an alkyl group having 1 to 18 carbon atoms, and may be the same as or different from each other.]

In general formula (1), examples of the alkyl group having 1 to 18 carbon atoms as each of R¹ to R⁴ include linear alkyl groups (methyl, ethyl, n-propyl, n-butyl, n-pentyl, n-octyl, n-decyl, n-dodecyl, n-tetradecyl, n-hexadecyl, n-octadecyl and the like), branched alkyl groups (isopropyl, isobutyl, sec-butyl, tert-butyl, isopentyl, neopentyl, tert-pentyl, isohexyl, 2-ethylhexyl, 1,1,3,3-tetramethylbutyl and the like), cycloalkyl groups (cyclopropyl, cyclobutyl, cyclopentyl, cyclohexyl and the like) and bridged cyclic alkyl groups (norbornyl, adamantyl, pinanyl and the like).

In general formula (1), examples of the aryl group having 6 to 14 carbon atoms (excluding carbon atoms contained in a substituent as mentioned below) as each of R¹ to R⁴ include monocyclic aryl groups (phenyl and the like), condensed polycyclic aryl groups (naphthyl, anthryl, phenanthryl, anthraquinonyl, fluorenyl, naphthoquinonyl and the like) and aromatic heterocyclic hydrocarbon groups (monocyclic heterocycles such as thienyl, furyl, pyranyl, pyrrolyl, oxazolyl, thiazolyl, pyridyl, pyrimidinyl and pyrazinyl; and condensed polycyclic heterocycles such as indolyl, benzofuranyl, isobenzofuranyl, benzothienyl, isobenzothienyl, quinolyl, isoquinolyl, quinoxalinyl, quinazolinyl, carbazolyl, acridinyl, phenothiazinyl, phenazinyl, xanthenyl, thianthrenyl, phenoxazinyl, phenoxathiinyl, chromanyl, isochromanyl, coumarinyl, dibenzothienyl, xanthonyl, thioxanthonyl and dibenzofuranyl).

Beside the groups listed above, examples of the aryl group may include aryl groups in which some of hydrogen atoms in the aryl groups are independently substituted by an alkyl group having 1 to 18 carbon atoms, an alkenyl group having 2 to 18 carbon atoms, an alkynyl group having 2 to 18 carbon atoms, an aryl group having 6 to 14 carbon atoms, a nitro group, a hydroxyl group, a cyano group, an alkoxy group or aryloxy group represented by -OR²¹, an acyl group represented by R²²CO-, an acyloxy group represented by R²³COO-, an alkylthio group or arylthio group represented by -SR²⁴, an amino group represented by -NR²⁵R²⁶, or a halogen atom.

In the above-mentioned substituent, examples of the alkenyl group having 2 to 18 carbon atoms include linear or branched alkenyl groups (vinyl, allyl, 1-propenyl, 2-propenyl, 1-butenyl, 2-butenyl, 3-butenyl, 1-methyl-1-propenyl, 1-methyl-2-propenyl, 2-methyl-1-propenyl, 2-methyl-2-propenyl and the like), cycloalkenyl groups (2-cyclohexenyl, 3-cyclohexenyl and the like) and arylalkenyl groups (styryl, cinnamyl and the like).

In the above-mentioned substituent, examples of the alkynyl group having 2 to 18 carbon atoms include linear or branched alkynyl groups (ethynyl, 1-propynyl, 2-propynyl, 1-butynyl, 2-butynyl, 3-butynyl, 1-methyl-2-propynyl, 1,1-dimethyl-2-propynyl, 1-pentynyl, 2-pentynyl, 3-pentynyl, 4-pentynyl, 1-methyl-2-butynyl, 3-methyl-1-butynyl, 1-decynyl, 2-decynyl, 8-decynyl, 1-dodecynyl, 2-dodecynyl, 10-dodecynyl and the like) and arylalkynyl groups (phenylethynyl and the like). Beside the groups listed above, examples of the alkynyl group may include substituted alkynyl groups in which some of hydrogen atoms in the alkynyl groups are independently substituted by a nitro group, a cyano group, a halogen atom, an alkoxy group having 1 to 18 carbon atoms and/or an alkylthio group having 1 to 18 carbon atoms, or the like.

In the above-mentioned substituent, examples of each of R²¹ to R²⁶ of an alkoxy group represented by -OR²¹, an acyl group represented by R²²CO-, an acyloxy group represented by R²³COO-, an alkylthio group represented by-SR²⁴, and an amino group represented by -NR²⁵R²⁶ include an alkyl group having 1 to 8 carbon atoms, and specifically, examples thereof include an alkyl group having 1 to 8 carbon atoms among the above-mentioned alkyl groups.

In the above-mentioned substituent, examples of each of R²¹ to R²⁶ of an aryloxy group represented by -OR²¹, an acyl group represented by R²²CO-, an acyloxy group represented by R²³COO-, an arylthio group represented by-SR²⁴, and an amino group represented by -NR²⁵R²⁶ include an aryl group having 6 to 14 carbon atoms, and specifically, examples thereof include the above-mentioned aryl groups having 6 to 14 carbon atoms.

Examples of the alkoxy group represented by -OR²¹ include methoxy, ethoxy, n-propoxy, iso-propoxy, n-butoxy, sec-butoxy, tert-butoxy, n-pentoxy, iso-pentoxy, neo-pentoxy, 2-methylbutoxy and the like.

Examples of the aryloxy group represented by -OR²¹ include phenoxy, naphthoxy and the like.

Examples of the acyl group represented by R²²CO-include acetyl, propanoyl, butanoyl, pivaloyl, benzoyl and the like.

Examples of the acyloxy group represented by R²³COO-include acetoxy, butanoyloxy, benzoyloxy and the like.

Examples of the alkylthio group represented by -SR²⁴ include methylthio, ethylthio, butylthio, hexylthio, cyclohexylthio and the like.

Examples of the arylthio group represented by -SR²⁴ include phenylthio, naphthylthio and the like.

Examples of the amino group represented by -NR²⁵R²⁶ include methylamino, ethylamino, propylamino, dimethylamino, diethylamino, methylethylamino, dipropylamino, piperidino and the like.

Examples of the halogen atom include a fluorine atom, a chlorine atom, a bromine atom, an iodine atom and the like.

E represents hydrogen atom or a group represented by general formula (5) below.

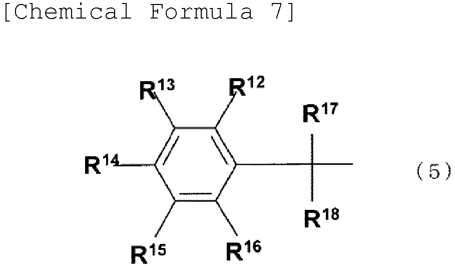

Among R¹² to R¹⁸, examples of the alkyl group having 1 to 18 carbon atoms, the alkoxy group represented by -OR²¹, the acyl group represented by R²²CO-, the acyloxy group represented by R²³COO-, the alkylthio group represented by -SR²⁴, or the amino group represented by -NR²⁵R²⁶ include those that are the same as said groups.

The ammonio group (Y⁺) leaves in the form of a corresponding amine (Y) with photoirradiation, and functions as various reaction catalysts. On the other hand, since the ammonio group (Y⁺) has no basicity before photoirradiation, the storage stability of a reactive composition is lowered even when the group is contained in the reactive composition.

The ammonio group (Y⁺) is represented by either general formula (2) or general formula (3) below.

Among R⁵ to R⁶ in general formula (2), the alkyl group having 1 to 18 carbon atoms is the same as the above-mentioned alkyl group, and the aryl group having 6 to 14 carbon atoms is the same as the above-mentioned aryl group. Moreover, these substituents may be bonded to each other to form a ring structure.

In general formula (2), Q represents a methylene group (-CH₂-)m, or a group represented by general formula (4) below, and m represents an integer of 2 or 3.

The substituents R¹⁰ to R¹¹ in general formula (4) independently represent a hydrogen atom, an alkyl group having 1 to 18 carbon atoms or an aryl group having 6 to 14 carbon atoms, the alkyl group having 1 to 18 carbon atoms is the same as the above-mentioned alkyl group, and the aryl group having 6 to 14 carbon atoms is the same as the above-mentioned aryl group. Moreover, these substituents may be bonded to each other to form a ring structure.

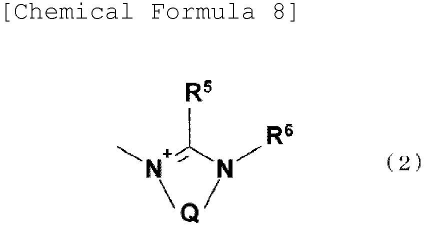

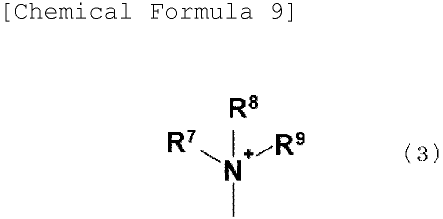

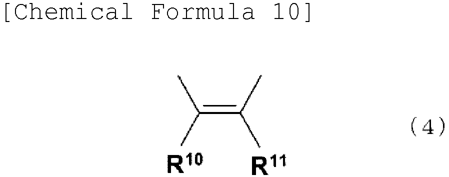

The substituents R⁷ to R⁹ in general formula (3) independently represent an alkyl group having 1 to 18 carbon atoms, an alkenyl group having 2 to 18 carbon atoms or an aryl group having 6 to 14 carbon atoms, and may be bonded to one another to form a ring structure. The alkyl group having 1 to 18 carbon atoms, alkenyl group having 2 to 18 carbon atoms and aryl group having 6 to 14 carbon atoms are the same as the above-mentioned alkyl group having 1 to 18 carbon atoms, alkenyl group having 2 to 18 carbon atoms and aryl group having 6 to 14 carbon atoms, respectively.

Examples of the ammonio group represented by general formula (2) include 1,8-diazabicyclo[5.4.0]undec-7-en-8-yl {a group represented by chemical formula (6)}, 1,5-diazabicyclo[4.3.0] non-5-en -5-yl {a group represented by chemical formula (7)}, and a group represented by general formula (8). Specifically, examples thereof include 1-methylimidazol-3-yl, 1,2-dimethylimidazol-3-yl, 1-methyl-2-ethylimidazol-3-yl and the like.

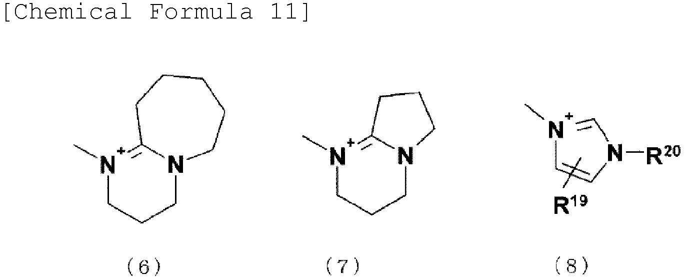

The substituent R²⁰ in general formula (8) represents an alkyl group having 1 to 18 carbon atoms, R¹⁹ represents an alkyl group having 1 to 18 carbon atoms, an alkenyl group having 2 to 18 carbon atoms or an aryl group having 6 to 14 carbon atoms, and these substituents may be bonded to each other to form a ring structure. Examples of the alkyl group having 1 to 18 carbon atoms include those that are the same as the alkyl groups mentioned above.

Examples of the alkenyl group having 2 to 18 carbon atoms include the above-mentioned alkenyl groups.

Examples of the aryl group having 6 to 14 carbon atoms include the above-mentioned aryl groups.

Examples of the ammonio group represented by general formula (3) include 1-azabicyclo[2.2.2]octan-1-yl {a group represented by chemical formula (9)}, 3-hydroxy-1-azabicyclo[2.2.2]octan-1-yl {a group represented by chemical formula (10)}, 1,4-diazabicyclo[2.2.2]octan-1-yl {a group represented by chemical formula (11)}, tributylammonio, trioctylammonio, octyldimethylammonio, diisopropyl ethylammonio and the like.

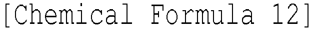

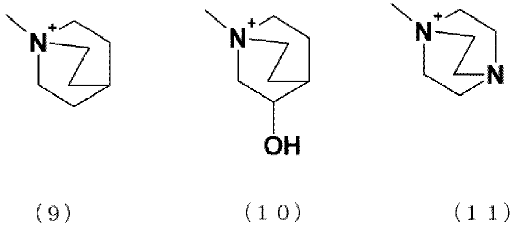

Among these ammonio groups, preferred are 1,8-diazabicyclo[5.4.0]undec-7-en-8-yl (a group represented by chemical formula (6)), 1,5-diazabicyclo[4.3.0] non-5-en -5-yl (a group represented by chemical formula (7)), 1-methylimidazol-3-yl, 1,2-dimethylimidazol-3-yl, 1-methyl-2-ethylimidazol-3-yl, 1-azabicyclo[2.2.2]octan-1-yl (a group represented by chemical formula (9)), 3-hydroxy-1-azabicyclo[2.2.2]octan-1-yl (a group represented by chemical formula (10)), 1,4-diazabicyclo[2.2.2]octan-1-yl (a group represented by chemical formula (11)), trioctylammonio and diisopropyl ethylammonio, and further preferred are 1,8-diazabicyclo[5.4.0]-7-undecen-8-yl (a group represented by chemical formula (6)), 1,5-diazabicyclo[5.4.0]-5-nonen-5-yl (a group represented by chemical formula (7)), 1-methylimidazol-3-yl and 1,2-dimethylimidazol-3-yl.

For example, the anion structure of a photobase generator represented by general formula (1) can be preferably exemplified by those represented by chemical formulas (A-1) to (A-8) below.

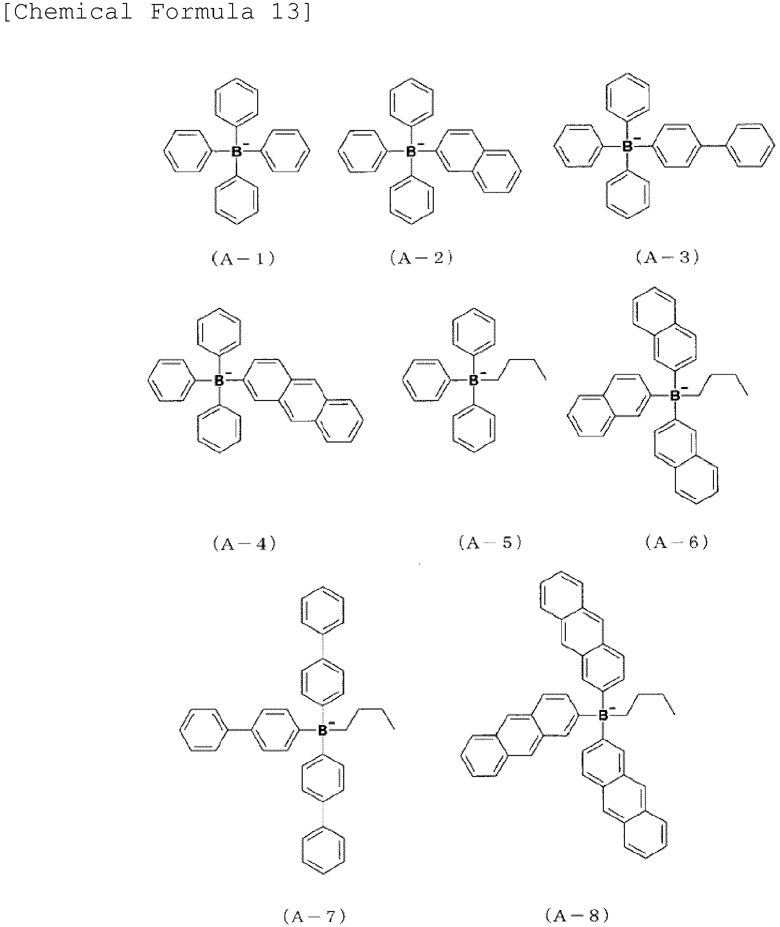

For example, the cation structure of a photobase generator represented by general formula (1) can be preferably exemplified by those represented by chemical formulas (CA-1) to (CA-9) below.

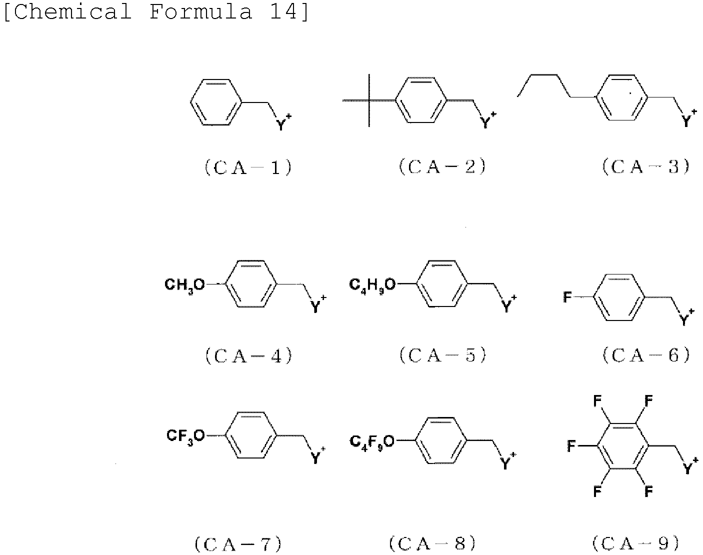

The photobase generator according to the present invention allows a photosensitizer to exert a higher effect compared with conventional photobase generators, and furthermore, the photocurability can be enhanced when used in combination with the photosensitizer.

As the photosensitizer, known (JP-A-11-279212, JP-A-09-183960 and the like) sensitizers can be used, and examples thereof include benzoquinones {1,4-benzoquinone, 1,2-benzoquinone and the like}; naphthoquinones {1,4-naphthoquinone, 1,2-naphthoquinone and the like}; anthraquinones {2-methyl anthraquinone, 2-ethyl anthraquinone and the like}; anthracenes {anthracene, 9,10-dibutoxyanthracene, 9,10-dimethoxyanthracene, 9,10-diethoxyanthracene, 2-ethyl-9,10-dimethoxyanthracene, 9,10-dipropoxyanthracene and the like}; pyrene; 1,2-benzanthracene; perylene; tetracene; coronene; thioxanthones {thioxanthone, 2-methylthioxanthone, 2-ethylthioxanthone, 2-chlorothioxanthone, 2-isopropylthioxanthone, 2,4-diethylthioxanthone and the like}; phenothiazines {phenothiazine, N-methylphenothiazine, N-ethylphenothiazine, N-phenylphenothiazine and the like}; xanthone; naphthalenes {1-naphthol, 2-naphthol, 1-methoxynaphthalene, 2-methoxynaphthalene, 1,4-dihydroxynaphthalene, 4-methoxy-1-naphthol and the like}; ketones {dimethoxyacetophenone, diethoxyacetophenone, 2-hydroxy-2-methyl-1-phenylpropan-1-one, 4'-isopropyl-2-hydroxy-2-methylpropiophenone, 4-benzoyl-4'-methyldiphenylsulfide and the like}; carbazole {N-phenylcarbazole, N-ethylcarbazole, poly-N-vinylcarbazole, N-glycidylcarbazole and the like}; chrysenes {1,4-dimethoxychrysene, 1,4-di-α-methylbenzyloxychrysene and the like}; phenanthrenes {9-hydroxyphenanthrene, 9-methoxyphenanthrene, 9-hydroxy-10-methoxyphenanthrene, 9-hydroxy-10-ethoxyphenanthrene and the like} and the like.

In particular, from the viewpoint of electron acceptability, a naphthoquinone-based, benzophenone-based, xanthone-based, anthraquinone-based or thioxanthone-based sensitizer is preferred because a high sensitizing effect is attained when the sensitizer is used.

The photobase generator according to the present invention can be produced by a known method. An example thereof is shown by the following chemical reaction formulas. By allowing a compound (G) having a substituent corresponding to an aimed photobase generator and a leaving group (Z) as a substituent and an amine (Y) corresponding to the ammonio group (Y⁺) to undergo a reaction directly or in a solvent, a cation intermediate containing Z⁻ as a counter anion is obtained. This cation intermediate and a borate metal salt which has a substituent corresponding to the aimed photobase generator and is separately produced by a known method can be subjected to an anion exchange in an organic solvent or water to obtain the aimed photobase generator.

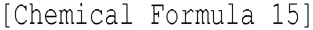

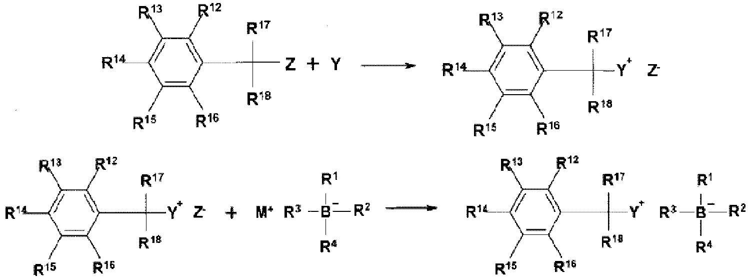

[In the formulas, R¹ to R⁴, R¹² to R¹⁸ and Y⁺ are the same as those in general formula (1), Z is a leaving group, Z⁻ is a counter anion generated through elimination, Y is an amine corresponding to ammonium, and M⁺ is a metal cation.]

Examples of the amine (Y) include an amine represented by chemical formula (12) {1,8-diazabicyclo[5,4,0]-undecene-7 (DBU; "DBU" is a registered trademark of San-Apro Ltd.)}, an amine represented by chemical formula (13) {1,5-diazabicyclo[4,3,0]-nonene-5 (DBN)}, amines represented by chemical formula (14) {each sign is the same as that of chemical formula (3). For example, cyclic amines such as 1-azabicyclo[2.2.2]octane, 3-hydroxy-1-azabicyclo[2.2.2]octane and 1,4-diazabicyclo[2.2.2]octane, chain amines such as trialkylamines (tributylamine, trioctylamine, octyldimethylamine, diisopropylethylamine and the like), trialkenylamines (triallylamine and the like) and triarylamines (triphenylamine, tri p-tolylamine, diphenyl p-tolylamine and the like) and the like}, and amines represented by chemical formula (15) {each sign is the same as that of chemical (8), for example, 1-methylimidazole, 1,2-dimethylimidazole and 1-methyl-2-ethylimidazole) and the like}.

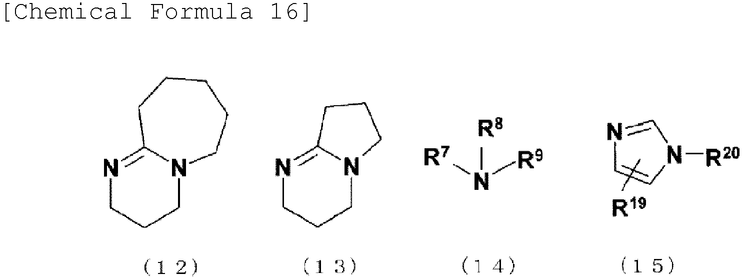

Examples of the leaving group (Z) include halogen atoms (a chlorine atom, a bromine atom and the like), sulfonyloxy groups (trifluoromethylsulfonyloxy, 4-methylphenylsulfonyloxy, methylsulfonyloxy and the like) and acyloxy (acetoxy, trifluoromethylcarbonyloxy and the like). Among these, from the viewpoint of production easiness, a halogen atom and a sulfonyloxy group are preferred.

As the solvent, water or an organic solvent can be used. Examples of the organic solvent include hydrocarbons (hexane, heptane, toluene, xylene and the like), cyclic ethers (tetrahydrofuran, dioxane and the like), chlorine-based solvents (chloroform, dichloromethane and the like), alcohols (methanol, ethanol, isopropyl alcohol and the like), ketones (acetone, methyl ethyl ketone, methyl isobutyl ketone and the like), nitriles (acetonitrile and the like), and polar organic solvents (dimethyl sulfoxide, dimethylformamide, N-methylpyrrolidone and the like). These solvents may be used alone and two or more kinds thereof may be used in combination.

The reaction temperature (°C) between the compound (G) as a raw material of the cation intermediate and the amine (Y) is preferably -10 to 100, and further preferably 0 to 80. It is preferred that the compound (G) be previously dissolved in the organic solvent and then the amine be added thereto. With regard to the procedure for adding the amine, the amine may be added dropwise, and may be added dropwise after being diluted with an organic solvent.

The above-mentioned compound (G) can be produced by a known method. The compound (G) can be obtained by halogenating (preferably, brominating) a carbon atom at the α-position having an aromatic ring group as a substituent. Although the halogenation (preferably, bromination) can be performed by various ways, a method in which halogen (preferably, bromine) is used or a method in which N-bromosuccinimide is used together with a radical generator is simple and convenient and therefore is preferable (The Fourth Series of Experimental Chemistry, Vol. 19, edited by the Chemical Society of Japan, p. 422).

The borate metal salt as an anion component is obtained by allowing an alkyl or aryl organic metal compound, and an alkyl or aryl boron compound or a halogenated boron compound to undergo a reaction in an organic solvent by means of a known method (for example, Journal of Polymer Science: Part A: Polymer Chemistry, Vol. 34, 2817 (1996), or the like serves as a useful reference). As the organic metal compound to be used, lithium compounds such as an alkyl lithium and an aryl lithium, and magnesium compounds (Grignard reagents) such as an alkyl magnesium halide and an aryl magnesium halide are suitably used.

The temperature in a reaction between the boron compound and the organic metal compound is -80°C to 100°C, preferably -50°C to 50°C, and most preferably -30°C to 30°C. As the organic solvent to be used, hydrocarbons (hexane, heptane, toluene, xylene and the like), cyclic ethers (tetrahydrofuran, dioxane and the like), and chlorine-based solvents (chloroform, dichloromethane and the like) are suitably used.

From the viewpoints of stability and solubility, it is preferred that the borate metal salt obtained as above be an alkali metal salt. In the case of allowing those to undergo a reaction with a Grignard reagent, it is preferred that sodium hydrogen carbonate, sodium chloride, potassium chloride, lithium chloride, sodium bromide, potassium bromide, lithium bromide and the like be added to perform the exchange of the metal during the reaction or after the reaction.

The anion exchange is performed by mixing the borate metal salt obtained as above and an organic solvent containing an intermediate or an aqueous solution thereof.

After obtaining an intermediate, anion exchange may be performed subsequently or, alternatively, anion exchange may be performed after isolating and purifying the intermediate and then dissolving it in an organic solvent again.

The photobase generator obtained as described above may be purified after being separate from the organic solvent. The separation from the organic solvent can be performed by adding a poor solvent to an organic solvent solution containing the photobase generator directly (or after the condensation thereof) to deposit the photobase generator. Examples of the poor solvent to be used include chain ethers (diethyl ether, dipropyl ether and the like), esters (ethyl acetate, butyl acetate and the like), aliphatic hydrocarbons (hexane, cyclohexane and the like) and aromatic hydrocarbons (toluene, xylene and the like).

When the photobase generator is an oily substance, the photobase generator according to the present invention can be obtained by separating the deposited oily substance from the organic solvent solution, and furthermore, distilling away the organic solvent contained in the oily substance. On the other hand, when the photobase generator is a solid, the photobase generator according to the present invention can be obtained by separating a deposited solid from the organic solvent solution, and furthermore, distilling away the organic solvent contained in the solid.

With regard to the purification, the photobase generator can be purified by recrystallization (a method of utilizing the solubility difference caused by cooling, a method of adding a poor solvent to deposit the photobase generator, and combined use of these methods). Moreover, when the photobase generator is an oily substance (when the photobase generator is not crystallized), the photobase generator can be purified by a method of washing the oily substance with water or a poor solvent.

The photobase generator according to the present invention can be applied as a latent base catalyst (a catalyst that has no catalysis before photoirradiation but develops an action of a base catalyst through photoirradiation) and the like, can be used as a curing catalyst for a basic reactive compound, for example, a photosensitive resin composition such as a photocurable resin composition, and is suitable as a curing catalyst for a photocurable resin composition which is cured when irradiated with light. For example, a photocurable resin composition containing a basic resin in which curing is promoted by a base, the photobase generator according to the present invention, and if necessary, a solvent and/or additive can be easily constituted. Since such a photocurable resin composition contains the photobase generator according to the present invention, the photocurable resin composition is excellent in curing properties as well as excellent in preservation stability. That is, it is possible to obtain a cured material by irradiating a photocurable resin composition containing the photobase generator according to the present invention with light having a wavelength of 350 to 500 nm to generate a base and promoting a curing reaction. Thus, a production method of such a cured material preferably includes a step of irradiating the photobase generator according to the present invention with light having a wavelength of 350 to 500 nm to generate a base. In the curing reaction, heating may be performed, if necessary.

The photosensitive resin composition in which curing is promoted by a base generated by photoirradiation is not particularly limited as long as the photosensitive resin composition is a photocurable resin cured by a base, and examples thereof include curable urethane resins {resins containing a (poly)isocyanate and a curing agent (a polyol, a thiol and the like) and the like}, curable epoxy resins {resins containing a (poly)epoxide and a curing agent (an acid anhydride, a carboxylic acid, a (poly)epoxide, a thiol and the like), resins containing epichlorohydrin and a carboxylic acid, and the like}, curable acrylic resins {an acrylic monomer and/or an acrylic oligomer and a curing agent (a thiol, a malonic ester, acetylacetonato and the like)}, polysiloxane (which is cured to form a crosslinked polysiloxane), polyimide resins, and resins described in Patent Document 3.

For the photobase generator according to the present invention, there can be used an ultra-high pressure mercury lamp, a metal halide lamp, a high power metal halide lamp and the like (Recent Advances in UV/EB Radiation Curing Technology, edited by RadTech Japan, published by CMC Publishing CO., LTD., p. 138, 2006) as well as a commonly used high pressure mercury lamp.

### EXAMPLES

Hereinafter, the present invention will be further described by reference to examples, but the present invention is not intended to be limited thereto. It should be noted that % means % by weight unless otherwise stated.

### Production Example 1 Synthesis of sodium 2-naphthyltriphenylborate

In a four-necked reaction vessel, the inside of which was replaced with nitrogen, 100 mL of a 0.25 molL-¹ tetrahydrofuran solution of triphenylborane (available from Aldrich) was placed, and cooled to -20°C. To the solution, 26 mL of a 1.0 molL-¹ solution of 2-naphthylmagnesium bromide prepared by a routine procedure from 2-bromonaphthalene was gradually added dropwise. After dropping, the contents were stirred for 2 hours at room temperature, after which to this solution, 100 ml of a saturated aqueous sodium bicarbonate solution was added, an organic layer was separated and subjected to solvent removal, and the residue was washed with hexane two times and then dried under reduced pressure to obtain an aimed product.

### Production Example 2 Synthesis of sodium 4-biphenylyltriphenylborate

In place of 2-bromonaphthalene, 4-bromobiphenyl was used to prepare an aimed product according to the method described in Production Example 1.

### Production Example 3 Synthesis of sodium 2-anthryltriphenylborate

In place of 2-bromonaphthalene, 2-bromoanthracene was used to prepare an aimed product according to the method described in Production Example 1.

### Production Example 4 Synthesis of sodium (4-methoxyphenyl)triphenylborate

In place of 2-bromonaphthalene, 4-bromoanisole was used to prepare an aimed product according to the method described in Production Example 1.

### Production Example 5 Synthesis of sodium (4-n-octylphenyl)triphenylborate

In place of 2-bromonaphthalene, 4-n-octylbromobenzene was used to prepare an aimed product according to the method described in Production Example 1.

### Production Example 6 Synthesis of lithium n-butyltriphenylborate

In a four-necked reaction vessel, 3.5 g (0.025 mol) of a boron trifluoride-ether complex was placed, and furthermore, 100 mL of tetrahydrofuran was placed and stirred. The contents were cooled to -20°C, and to the contents, 74 mL of a 1.0 molL⁻¹ solution of phenylmagnesium bromide prepared by a routine procedure from bromobenzene was gradually added dropwise. The formation of triphenylborane and the completion of the reaction were confirmed by NMR, after which 12 mL of a 2.0 molL-¹ cyclohexane solution of n-butyllithium (available from Aldrich) was added dropwise. After dropping, the contents were stirred for 2 hours at room temperature, were added with 100 mL of hexane to perform filtration, and were dried under reduced pressure to obtain an aimed product.

### Production Example 7 Synthesis of lithium n-butyltri(2-naphthyl)borate

In place of bromobenzene, 2-bromonaphthalene was used to prepare an aimed product according to the method described in Production Example 6.

### Production Example 8 Synthesis of lithium n-butyltri(4-biphenylyl)borate

In place of bromobenzene, 4-bromobiphenyl was used to prepare an aimed product according to the method described in Production Example 6.

### Production Example 9 Synthesis of lithium n-butyltri(2-anthryl)borate

In place of bromobenzene, 2-bromoanthracene was used to prepare an aimed product according to the method described in Production Example 6.

### Production Example 10 Synthesis of lithium n-butyltri(4-fluorophenyl)borate

In place of bromobenzene, p-bromofluorobenzene was used to prepare an aimed product according to the method described in Production Example 6.

### Production Example 11 Synthesis of sodium benzyltriphenylborate

Instead of preparing a 1.0 molL-¹ solution of 2-naphthylmagnesium bromide, a 1.0 molL-¹ solution of benzylmagnesium chloride (available from Aldrich) was used to prepare an aimed product according to the method described in Production Example 1.

### Example 1 Synthesis of Compound B-1

### (1) Synthesis of Intermediate (a)

In 100 g of chloroform, 23 g of benzyl chloride was dissolved, and to this, 27 g of 1,8-diazabicyclo[5.4.0] undec-7-ene (DBU, available from San-Apro Ltd.) was added dropwise. The contents were stirred at room temperature. After 2 hours, the disappearance of the raw materials was confirmed by HPLC, and a 50% chloroform solution of Intermediate (a) was obtained.

### (2) Synthesis of Compound B-1

To 10 g of the 50% chloroform solution of Intermediate (a) obtained in (1), 6.1 g of sodium tetraphenylborate (available from NACALAI TESQUE, INC.) and 50 g of ion-exchanged water were added and stirred for 3 hours at room temperature. An organic layer was washed with 50 g of ion-exchanged water three times. The organic layer was concentrated and the solvent was evaporated, after which the residue was subjected to silica gel chromatography to obtain a white solid. It was confirmed by 1H-NMR that this white solid was Compound B-1. The structure of Compound B-1 was described in Table 1.

### Example 2 Synthesis of Compound B-2

In place of 6.1 g of sodium tetraphenylborate (available from NACALAI TESQUE, INC.), 7.0 g of sodium 2-naphthyltriphenylborate obtained in Production Example 1 was used to prepare an aimed product according to the method described in Example 1. The structure of Compound B-2 was described in Table 1.

### Example 3 Synthesis of Compound B-3

In place of 6.1 g of sodium tetraphenylborate (available from NACALAI TESQUE, INC.), 9.3 g of sodium 4-biphenylyltriphenylborate obtained in Production Example 2 was used to prepare an aimed product according to the method described in Example 1. The structure of Compound B-3 was described in Table 1.

### Example 4 Synthesis of Compound B-4

In place of 6.1 g of sodium tetraphenylborate (available from NACALAI TESQUE, INC.), 10.7 g of sodium 2-anthryltriphenylborate obtained in Production Example 3 was used to prepare an aimed product according to the method described in Example 1. The structure of Compound B-4 was described in Table 1.

### Example 5 Synthesis of Compound B-5

In place of 6.1 g of sodium tetraphenylborate (available from NACALAI TESQUE, INC.), 5.9 g of lithium n-butyltriphenylborate obtained in Production Example 6 was used to prepare an aimed product according to the method described in Example 1. The structure of Compound B-5 was described in Table 1.

### Example 6 Synthesis of Compound B-6

In place of 6.1 g of sodium tetraphenylborate (available from NACALAI TESQUE, INC.), 8.6 g of lithium n-butyltri(2-naphthyl)borate obtained in Production Example 7 was used to prepare an aimed product according to the method described in Example 1. The structure of Compound B-6 was described in Table 1.

### Example 7 Synthesis of Compound B-7

In place of 6.1 g of sodium tetraphenylborate (available from NACALAI TESQUE, INC.), 10.0 g of lithium n-butyltri(4-biphenylyl)borate obtained in Production Example 8 was used to prepare an aimed product according to the method described in Example 1. The structure of Compound B-7 was described in Table 1.

### Example 8 Synthesis of Compound B-8

In place of 6.1 g of sodium tetraphenylborate (available from NACALAI TESQUE, INC.), 11.3 g of lithium n-butyltri(2-anthryl)borate obtained in Production Example 9 was used to prepare an aimed product according to the method described in Example 1. The structure of Compound B-8 was described in Table 1.

### Example 9 Synthesis of Compound B-9

In place of 6.1 g of sodium tetraphenylborate (available from NACALAI TESQUE, INC.), 6.6 g of sodium (4-methoxyphenyl)triphenylborate obtained in Production Example 4 was used to prepare an aimed product according to the method described in Example 1. The structure of Compound B-9 was described in Table 1.

### Example 10 Synthesis of Compound B-10

In place of 6.1 g of sodium tetraphenylborate (available from NACALAI TESQUE, INC.), 8.1 g of sodium (4-n-octylphenyl)triphenylborate obtained in Production Example 5 was used to prepare an aimed product according to the method described in Example 1. The structure of Compound B-10 was described in Table 1.

### Example 11 Synthesis of Compound B-11

In place of 6.1 g of sodium tetraphenylborate (available from NACALAI TESQUE, INC.), 6.4 g of lithium n-butyltri(4-fluorophenyl)borate obtained in Production Example 10 was used to prepare an aimed product according to the method described in Example 1. The structure of Compound B-11 was described in Table 1.

### Example 12 Synthesis of Compound B-12

In place of 6.1 g of sodium tetraphenylborate (available from NACALAI TESQUE, INC.), 6.3 g of sodium benzyltriphenylborate obtained in Production Example 11 was used to prepare an aimed product according to the method described in Example 1. The structure of Compound B-12 was described in Table 1.

### Example 13 Synthesis of Compound B-13

### (1) Synthesis of Intermediate (b)

In 100 g of chloroform, 30 g of benzyl chloride was dissolved, and to this, 20 g of 1-methylimidazole was added dropwise. The contents were stirred at room temperature. After 5 hours, the disappearance of the raw materials was confirmed by HPLC, and a 50% chloroform solution of Intermediate (b) was obtained.

### (2) Synthesis of Compound B-13

To 6.3 g of the 50% chloroform solution of Intermediate (b) obtained in (1), 6.1 g of sodium tetraphenylborate (available from NACALAI TESQUE, INC.) and 50 g of ion-exchanged water were added and stirred for 3 hours at room temperature. An organic layer was washed with 50 g of ion-exchanged water three times. The organic layer was concentrated and the solvent was evaporated, after which the residue was subjected to silica gel chromatography to obtain a white solid. It was confirmed by 1H-NMR that this white solid was Compound B-13. The structure of Compound B-13 was described in Table 1.

### Example 14 Synthesis of Compound B-14

In place of 6.1 g of sodium tetraphenylborate (available from NACALAI TESQUE, INC.), 7.0 g of sodium 2-naphthyltriphenylborate obtained in Production Example 1 was used to prepare an aimed product according to the method described in Example 13. The structure of Compound B-14 was described in Table 1.

### Example 15 Synthesis of Compound B-15

In place of 6.1 g of sodium tetraphenylborate (available from NACALAI TESQUE, INC.), 9.3 g of sodium 4-biphenylyltriphenylborate obtained in Production Example 2 was used to prepare an aimed product according to the method described in Example 13. The structure of Compound B-15 was described in Table 1.

### Example 16 Synthesis of Compound B-16

In place of 6.1 g of sodium tetraphenylborate (available from NACALAI TESQUE, INC.), 10.7 g of sodium 2-anthryltriphenylborate obtained in Production Example 3 was used to prepare an aimed product according to the method described in Example 13. The structure of Compound B-16 was described in Table 1.

### Example 17 Synthesis of Compound B-17

In place of 6.1 g of sodium tetraphenylborate (available from NACALAI TESQUE, INC.), 5.9 g of lithium n-butyltriphenylborate obtained in Production Example 6 was used to prepare an aimed product according to the method described in Example 13. The structure of Compound B-17 was described in Table 1.

### Example 18 Synthesis of Compound B-18

In place of 6.1 g of sodium tetraphenylborate (available from NACALAI TESQUE, INC.), 8.6 g of lithium n-butyltri(2-naphthyl)borate obtained in Production Example 7 was used to prepare an aimed product according to the method described in Example 13. The structure of Compound B-18 was described in Table 1.

### Example 19 Synthesis of Compound B-19

In place of 6.1 g of sodium tetraphenylborate (available from NACALAI TESQUE, INC.), 10.0 g of lithium n-butyltri(4-biphenylyl)borate obtained in Production Example 8 was used to prepare an aimed product according to the method described in Example 13. The structure of Compound B-19 was described in Table 1.

### Example 20 Synthesis of Compound B-20

In place of 6.1 g of sodium tetraphenylborate (available from NACALAI TESQUE, INC.), 11.3 g of lithium n-butyltri(2-anthryl)borate obtained in Production Example 9 was used to prepare an aimed product according to the method described in Example 13. The structure of Compound B-20 was described in Table 1.

### Example 21 Synthesis of Compound B-21

In place of 6.1 g of sodium tetraphenylborate (available from NACALAI TESQUE, INC.), 6.6 g of sodium (4-methoxyphenyl)triphenylborate obtained in Production Example 4 was used to prepare an aimed product according to the method described in Example 13. The structure of Compound B-21 was described in Table 1.

### Example 22 Synthesis of Compound B-22

In place of 6.1 g of sodium tetraphenylborate (available from NACALAI TESQUE, INC.), 8.1 g of sodium (4-n-octylphenyl)triphenylborate obtained in Production Example 5 was used to prepare an aimed product according to the method described in Example 13. The structure of Compound B-22 was described in Table 1.

### Example 23 Synthesis of Compound B-23

In place of 6.1 g of sodium tetraphenylborate (available from NACALAI TESQUE, INC.), 6.4 g of lithium n-butyltri(4-fluorophenyl)borate obtained in Production Example 10 was used to prepare an aimed product according to the method described in Example 13. The structure of Compound B-23 was described in Table 1.

### Example 24 Synthesis of Compound B-24

In place of 6.1 g of sodium tetraphenylborate (available from NACALAI TESQUE, INC.), 6.3 g of sodium benzyltriphenylborate obtained in Production Example 11 was used to prepare an aimed product according to the method described in Example 13. The structure of Compound B-24 was described in Table 1.

**[Table 1]**

| | Compound | Y+ | Ar | R |
|---|---|---|---|---|
| Example 1 | B-1 | DBU | Phenyl | Phenyl |
| Example 2 | B-2 | DBU | Phenyl | 2-Naphthyl |
| Example 3 | B-3 | DBU | Phenyl | 4-Biphenyl |
| Example 4 | B-4 | DBU | Phenyl | 2-anthryl |
| Example 5 | B-5 | DBU | Phenyl | n-Butyl |
| Example 6 | B-6 | DBU | 2-Naphthyl | n-Butyl |
| Example 7 | B-7 | DBU | 4-Biphenyl | n-Butyl |
| Example 8 | B-8 | DBU | 2-anthryl | n-Butyl |
| Example 9 | B-9 | DBU | Phenyl | 4-Methoxyphenyl |
| Example 10 | B-10 | DBU | Phenyl | 4-n-Octylphenyl |
| Example 11 | B-11 | DBU | 4-Fluorophenyl | n-Butyl |
| Example 12 | B-12 | DBU | Phenyl | Benzyl |
| Example 13 | B-13 | 1-Methylimidazole | Phenyl | Phenyl |
| Example 14 | B-14 | 1-Methylimidazole | Phenyl | 2-Naphthyl |
| Example 15 | B-15 | 1-Methylimidazole | Phenyl | 4-Biphenyl |
| Example 16 | B-16 | 1-Methylimidazole | Phenyl | 2-anthryl |
| Example 17 | B-17 | 1-Methylimidazole | Phenyl | n-Butyl |
| Example 18 | B-18 | 1-Methylimidazole | 2-Naphthyl | n-Butyl |
| Example 19 | B-19 | 1-Methylimidazole | 4-Biphenyl | n-Butyl |
| Example 20 | B-20 | 1-Methylimidazole | 2-anthryl | n-Butyl |
| Example 21 | B-21 | 1-Methylimidazole | Phenyl | 4-Methoxyphenyl |
| Example 22 | B-22 | 1-Methylimidazole | Phenyl | 4-n-Octylphenyl |
| Example 23 | B-23 | 1-Methylimidazole | 4-Fluorophenyl | n-Butyl |
| Example 24 | B-24 | 1-Methylimidazole | Phenyl | Benzyl |

Y⁺, Ar and R in Table 1 denote symbols in the following general formula.

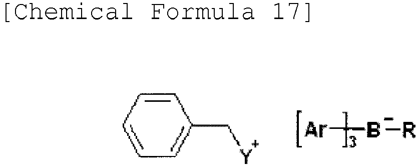

### Comparative Example 1 Synthesis of Photobase generator (H-1) below

According to the method described in Patent Document 7 (WO 2009/122664 A), an aimed product was synthesized.

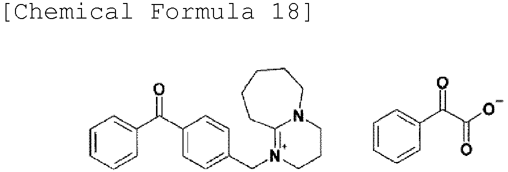

### Comparative Example 2 Synthesis of Photobase generator (H-2) below

According to the method described in Patent Document 7 (WO 2009/122664 A), an aimed product was synthesized.

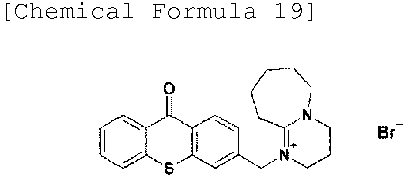

### Comparative Example 3 Synthesis of Photobase generator (H-3) below

According to the method described in Patent Document 7 (WO 2009/122664 A), an aimed product was synthesized.

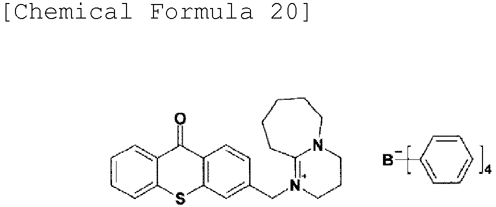

### Comparative Example 4 Synthesis of Photobase generator (H-4) below

Based on the method described in Patent Document 7 (WO 2009/122664 A), an aimed product was synthesized.

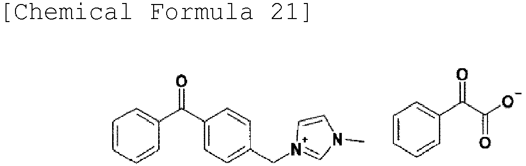

### Comparative Example 5 Synthesis of Photobase generator (H-5) below

Based on the method described in Patent Document 7 (WO 2009/122664 A), an aimed product was synthesized.

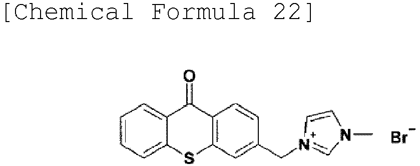

### Comparative Example 6 Synthesis of Photobase generator (H-6) below

Based on the method described in Patent Document 7 (WO 2009/122664 A), an aimed product was synthesized.

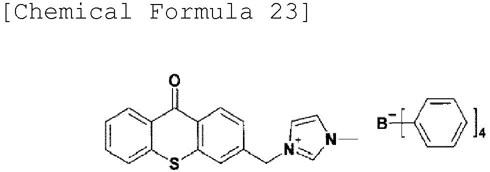

### Examples 25 to 36, Comparative Examples 1 to 3

By uniformly mixing 10 g of a bisphenol A type epoxy resin (Epikote 828, available from Mitsubishi Chemical Corporation), 9 g of an acid anhydride (HN5500E, available from Hitachi Chemical Company, Ltd.) and 0.5 g of a photobase generator, a mixture was prepared. The mixture was coated on a glass substrate (76 mm x 52 mm) by means of an applicator (40 µm) and then subjected to exposure with a belt conveyor type UV irradiation device (EYE GRAPHICS Co., Ltd. ECS-151U) to generate a base, immediately after which subsequently, the glass substrate was placed on a hot plate heated to 120°C and the time required for the tackiness of the coated surface to be eliminated was measured. These results were shown in Table 2.

**[Table 2]**

| | Photobase generator | Tack-free time (min) |
|---|---|---|
| Example 25 | B-1 | 100 |
| Example 26 | B-2 | 90 |
| Example 27 | B-3 | 100 |
| Example 28 | B-4 | 50 |
| Example 29 | B-5 | 80 |
| Example 30 | B-6 | 75 |
| Example 31 | B-7 | 85 |
| Example 32 | B-8 | 40 |
| Example 33 | B-9 | 95 |
| Example 34 | B-10 | 100 |
| Example 35 | B-11 | 85 |
| Example 36 | B-12 | 75 |
| Comparative Example 1 | H-1 | 120< |
| Comparative Example 2 | H-2 | Not cured |
| Comparative Example 3 | H-3 | 90 |

### Examples 37 to 72, Comparative Examples 4 to 12

By uniformly mixing 10 g of a bisphenol A type epoxy resin (Epikote 828, available from Mitsubishi Chemical Corporation), 9 g of an acid anhydride (HN5500E, available from Hitachi Chemical Company, Ltd.), 0.5 g of a photobase generator and 0.25 g of a photosensitizer (one with an outlined circle in Table 3 was used), a curable composition was prepared. The photobase generator used and the kind of the photosensitizer were described in Table 3.

This curable composition was coated on a glass substrate (76 mm × 52 mm) by means of an applicator (40 µm) and then subjected to exposure with a belt conveyor type UV irradiation device (EYE GRAPHICS Co., Ltd. ECS-151U) to generate a base, immediately after which subsequently, the glass substrate was placed on a hot plate heated to 120°C and the time required for the tackiness of the coated surface to be eliminated was measured. These results were shown in Table 3.

**[Table 3]**

| | Photobase generator | Photosensitizer | | | Tack-free time(min) |
|---|---|---|---|---|---|
| | | Benzophenone | Anthraquinone | Thioxanthone | |
| Example 37 | B-1 | ○ | | | 90 |
| Example 38 | B-2 | ○ | | | 85 |
| Example 39 | B-3 | ○ | | | 80 |
| Example 40 | B-4 | ○ | | | 45 |
| Example 41 | B-5 | ○ | | | 75 |
| Example 42 | B-6 | ○ | | | 65 |
| Example 43 | B-7 | ○ | | | 70 |
| Example 44 | B-8 | ○ | | | 35 |
| Example 45 | B-9 | ○ | | | 85 |
| Example 46 | B-10 | ○ | | | 90 |
| Example 47 | B-11 | ○ | | | 75 |
| Example 48 | B-12 | ○ | | | 65 |
| Example 49 | B-1 | | ○ | | 70 |
| Example 50 | B-2 | | ○ | | 60 |
| Example 51 | B-3 | | ○ | | 60 |
| Example 52 | B-4 | | ○ | | 40 |
| Example 53 | B-5 | | ○ | | 60 |
| Example 54 | B-6 | | ○ | | 65 |
| Example 55 | B-7 | | ○ | | 60 |
| Example 56 | B-8 | | ○ | | 20 |
| Example 57 | B-9 | | ○ | | 65 |
| Example 58 | B-10 | | ○ | | 70 |
| Example 59 | B-11 | | ○ | | 55 |
| Example 60 | B-12 | | ○ | | 45 |
| Example 61 | B-1 | | | ○ | 60 |
| Example 62 | B-2 | | | ○ | 55 |
| Example 63 | B-3 | | | ○ | 50 |
| Example 64 | B-4 | | | ○ | 30 |
| Example 65 | B-5 | | | ○ | 50 |
| Example 66 | B-6 | | | ○ | 40 |
| Example 67 | B-7 | | | ○ | 45 |
| Example 68 | B-8 | | | ○ | 20 |
| Example 69 | B-9 | | | ○ | 55 |
| Example 70 | B-10 | | | ○ | 60 |
| Example 71 | B-11 | | | ○ | 45 |
| Example 72 | B-12 | | | ○ | 35 |
| Comparative Example 4 | H-1 | ○ | | | 120 < |
| Comparative Example 5 | H-2 | ○ | | | Not cured |
| Comparative Example 6 | H-3 | ○ | | | 90 |
| Comparative Example 7 | H-1 | | ○ | | 120 < |
| Comparative Example 8 | H-2 | | ○ | | Not cured |
| Comparative Example 9 | H-3 | | ○ | | 85 |
| Comparative Example 10 | H-1 | | | ○ | 120 < |
| Comparative Example 11 | H-2 | | | ○ | Not cured |
| Comparative Example 12 | H-3 | | | ○ | 90 |

### Examples 73 to 120, Comparative Examples 13 to 24

By uniformly mixing 10 g of a bisphenol A type epoxy resin (Epikote 828, available from Mitsubishi Chemical Corporation), 0.5 g of a photobase generator and 0.25 g of a photosensitizer (one with an outlined circle in Table 3 was used, provided that no photosensitizer was added in Examples 73 to 84 and Comparative Examples 13 to 15), a curable composition was prepared. The photobase generator used and the kind of the photosensitizer were described in Table 3.

This curable composition was coated on a glass substrate (76 mm x 52 mm) by means of an applicator (40 µm) and then subjected to exposure with a belt conveyor type UV irradiation device (EYE GRAPHICS Co., Ltd. ECS-151U) to generate a base, and the gel time at 150°C was measured in accordance with the procedure of JISK5909. These results were shown in Tables 4 and 5.

**[Table 4]**

| | Photobase generator | Photosensitizer | | | Gel time (Sec) |
|---|---|---|---|---|---|
| | | Benzophenone | Anthraquinone | Thioxanthone | |
| Example 73 | B-13 | | | | 410 |
| Example 74 | B-14 | | | | 380 |
| Example 75 | B-15 | | | | 430 |
| Example 76 | B-16 | | | | 200 |
| Example 77 | B-17 | | | | 330 |
| Example 78 | B-18 | | | | 320 |
| Example 79 | B-19 | | | | 360 |
| Example 80 | B-20 | | | | 160 |
| Example 81 | B-21 | | | | 400 |
| Example 82 | B-22 | | | | 430 |
| Example 83 | B-23 | | | | 360 |
| Example 84 | B-24 | | | | 320 |
| Example 85 | B-13 | ○ | | | 360 |
| Example 86 | B-14 | ○ | | | 310 |
| Example 87 | B-15 | ○ | | | 350 |
| Example 88 | B-16 | ○ | | | 180 |
| Example 89 | B-17 | ○ | | | 270 |
| Example 90 | B-18 | ○ | | | 250 |
| Example 91 | B-19 | ○ | | | 290 |
| Example 92 | B-20 | ○ | | | 140 |
| Example 93 | B-21 | ○ | | | 310 |
| Example 94 | B-22 | ○ | | | 350 |
| Example 95 | B-23 | ○ | | | 260 |
| Example 96 | B-24 | ○ | | | 220 |
| Example 97 | B-13 | | ○ | | 310 |
| Example 98 | B-14 | | ○ | | 290 |
| Example 99 | B-15 | | ○ | | 330 |
| Example 100 | B-16 | | ○ | | 160 |
| Example 101 | B-17 | | ○ | | 260 |
| Example 102 | B-18 | | ○ | | 230 |
| Example 103 | B-19 | | ○ | | 280 |
| Example 104 | B-20 | | ○ | | 130 |

**[Table 5]**

| | Photobase generator | Photosensitizer | | | Gel time (Sec) |
|---|---|---|---|---|---|
| | | Benzophenone | Anthraquinone | Thioxanthone | |
| Example 105 | B-21 | | ○ | | 260 |
| Example 106 | B-22 | | ○ | | 300 |
| Example 107 | B-23 | | ○ | | 210 |
| Example 108 | B-24 | | ○ | | 170 |
| Example 109 | B-13 | | | ○ | 270 |
| Example 110 | B-14 | | | ○ | 230 |
| Example 111 | B-15 | | | ○ | 260 |
| Example 112 | B-16 | | | ○ | 140 |
| Example 113 | B-17 | | | ○ | 210 |
| Example 114 | B-18 | | | ○ | 190 |
| Example 115 | B-19 | | | ○ | 220 |
| Example 116 | B-20 | | | ○ | 100 |
| Example 117 | B-21 | | | ○ | 230 |
| Example 118 | B-22 | | | ○ | 270 |
| Example 119 | B-23 | | | ○ | 170 |
| Example 120 | B-24 | | | ○ | 140 |
| Comparative Example 13 | H-4 | | | | 600 |
| Comparative Example 14 | H-5 | | | | Not gelated |
| Comparative Example 15 | H-6 | | | | 400 |
| Comparative Example 16 | H-4 | ○ | | | 600 |
| Comparative Example 17 | H-5 | ○ | | | Not gelated |
| Comparative Example 18 | H-6 | ○ | | | 410 |
| Comparative Example 19 | H-4 | | ○ | | 550 |
| Comparative Example 20 | H-5 | | ○ | | Not gelated |
| Comparative Example 21 | H-6 | | ○ | | 370 |
| Comparative Example 22 | H-4 | | | ○ | 590 |
| Comparative Example 23 | H-5 | | | ○ | Not gelated |
| Comparative Example 24 | H-6 | | | ○ | 390 |

The results in Tables 2 to 5 reveal that the photobase generator according to the present invention has higher sensitivity to light compared with photobase generators for comparison, can also exert a high effect when used in combination with a photosensitizer, and thus is useful as a photobase generator.

## Claims

1. A photobase generator, comprising an ammonium salt represented by general formula (1): 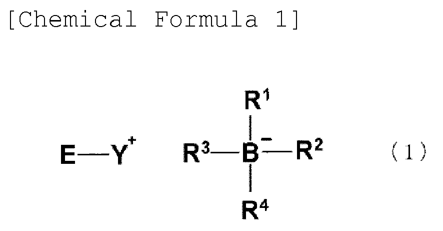 [In formula (1), R¹ to R⁴ independently represent an alkyl group having 1 to 18 carbon atoms or Ar, wherein at least one of R¹ to R⁴ represents Ar, Ar represents an aryl group having 6 to 14 carbon atoms (excluding carbon atoms contained in a substituent as mentioned below), some of hydrogen atoms in the aryl group may be independently substituted by an alkyl group having 1 to 18 carbon atoms, an alkenyl group having 2 to 18 carbon atoms, an alkynyl group having 2 to 18 carbon atoms, an aryl group having 6 to 14 carbon atoms, a nitro group, a hydroxyl group, a cyano group, an alkoxy group or aryloxy group represented by -OR²¹, an acyl group represented by R²²CO- , an acyloxy group represented by R²³COO-, an alkylthio group or arylthio group represented by -SR²⁴, an amino group represented by -NR²⁵R²⁶, or a halogen atom, R²¹ to R²⁴ independently represent an alkyl group having 1 to 8 carbon atoms or an aryl group having 6 to 14 carbon atoms, and R²⁵ and R²⁶ independently represent a hydrogen atom, an alkyl group having 1 to 8 carbon atoms or an aryl group having 6 to 14 carbon atoms; Y⁺ represents an ammonio group represented by general formula (2) or (3) below; in formula (2), R⁵ and R⁶ independently represent an alkyl group having 1 to 18 carbon atoms, an alkenyl group having 2 to 18 carbon atoms or an aryl group having 6 to 14 carbon atoms, and may be bonded to each other to form a ring structure; Q represents a methylene group (-CH₂-)m, or a group represented by general formula (4) below, and m represents an integer of 2 or 3; in formula (3), R⁷ to R⁹ independently represent an alkyl group having 1 to 18 carbon atoms, an alkenyl group having 2 to 18 carbon atoms or an aryl group having 6 to 14 carbon atoms, and may be bonded to one another to have a ring structure; in formula (4), R¹⁰ to R¹¹ independently represent a hydrogen atom, an alkyl group having 1 to 18 carbon atoms or an aryl group having 6 to 14 carbon atoms, and may be bonded to each other to form a ring structure; and E represents a hydrogen atom or a group represented by general formula (5) below, R¹² to R¹⁰ independently represent an alkyl group having 1 to 18 carbon atoms, a nitro group, a hydroxyl group, a cyano group, an alkoxy group represented by -OR²¹, an acyl group represented by R²²CO-, an acyloxy group represented by R²³COO-, an alkylthio group represented by -SR²⁴, an amino group represented by -NR²⁵R²⁶ or a halogen atom, R¹² to R¹⁶ may be the same as or different from one another, and R¹⁷ and R¹⁸ independently represent a hydrogen atom or an alkyl group having 1 to 18 carbon atoms, and may be the same as or different from each other.]

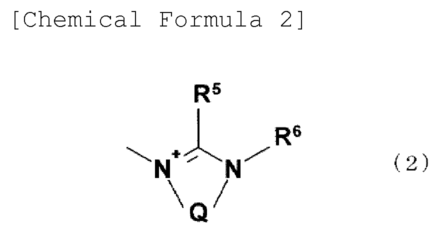

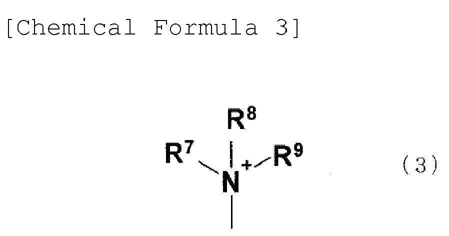

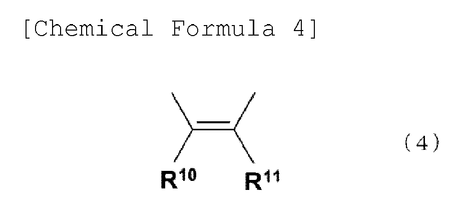

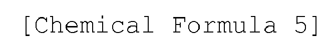

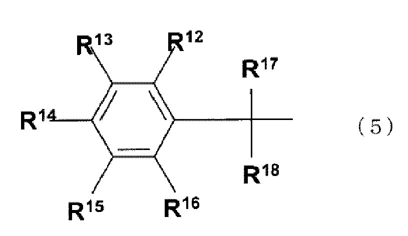

2. The photobase generator according to claim 1, further comprising a photosensitizer as an active ingredient.

3. The photobase generator according to claim 2, wherein the photosensitizer is an aromatic ketone.

4. The photobase generator according to any one of claims 1 to 3, wherein Ar of R¹ to R⁴ in general formula (1) represents a phenyl group, a naphthyl group, a biphenylyl group or an anthryl group.

5. The photobase generator according to any one of claims 1 to 3, wherein Y⁺ in general formula (1) represents an ammonio group represented by general formula (2).

6. The photobase generator according to claim 5, wherein Y⁺ in general formula (1) represents an ammonio group selected from the group consisting of general formulas (6) to (8) below: 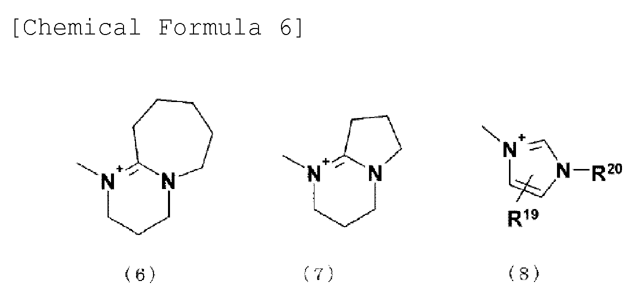 [In formula (8), R¹⁹ represents an alkyl group having 1 to 18 carbon atoms, R²⁰ represents an alkyl group having 1 to 18 carbon atoms, an alkenyl group having 2 to 18 carbon atoms or an aryl group having 6 to 14 carbon atoms, and R¹⁹ and R²⁰ may be bonded to each other to form a ring structure.].

7. A photocurable composition, comprising the photobase generator according to any one of claims 1 to 6 and a basic reactive compound.

8. A cured product obtained by curing the photocurable composition according to claim 7.
